(19) Europäisches Patentamt
European Patent Office
Office européen des brevets

(11) Veröffentlichungsnummer : **0 324 815 B1**

(12) # EUROPÄISCHE PATENTSCHRIFT

(45) Veröffentlichungstag der Patentschrift : **29.01.92 Patentblatt 92/05**

(51) Int. Cl.$^5$ : **C07C 405/00,** C07C 247/00, C07C 59/46, A61K 31/557

(21) Anmeldenummer : **88905719.6**

(22) Anmeldetag : **21.07.88**

(86) Internationale Anmeldenummer : **PCT/DE88/00458**

(87) Internationale Veröffentlichungsnummer : **WO 89/00990 09.02.89 Gazette 89/04**

(54) **NEUE 9-SUBSTITUIERTE CARBACYCLINDERIVATE, VERFAHREN ZU IHRER HERSTELLUNG UND IHRE VERWENDUNG ALS ARZNEIMITTEL.**

(30) Priorität : **24.07.87 DE 3725031**

(43) Veröffentlichungstag der Anmeldung : **26.07.89 Patentblatt 89/30**

(45) Bekanntmachung des Hinweises auf die Patenterteilung : **29.01.92 Patentblatt 92/05**

(84) Benannte Vertragsstaaten : **AT BE CH DE FR GB IT LI LU NL SE**

(56) Entgegenhaltungen :
**EP-A- 0 086 611**
**EP-A-02 242 75**
**DE-A-34 282 66**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **VORBRÜGGEN, Helmut**
**Wilkestrasse 7**
**W-1000 Berlin 27 (DE)**
Erfinder : **KLAR, Ulrich**
**Helmkrautstrasse 21**
**W-1000 Berlin 27 (DE)**
Erfinder : **NIEUWEBOER, Bob**
**Bärbelweg 16A**
**W-1000 Berlin 27 (DE)**
Erfinder : **STÜRZEBECHER, Claus-Steffen**
**Sponholzstrasse 34**
**W-1000 Berlin 41 (DE)**

EP 0 324 815 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue 9-substituierte Carbacyclin-Derivate, Verfahren zu ihrer Herstellung sowie ihre Verwendung als Arzneimittel.

In der US-Patentschrift 4,420,632 werden 9-alkylierte Carbacyclin-Derivate beschrieben, die antithrombotische, antisekretorische und bronchodilatierende Eigenschaften besitzen. Sie wirken außerdem thrombozytenaggregationshemmend.

Es wurde gefunden, daß sich in 9-Stellung kettenverlängerte Carbacyclin-Analoga mit einer reaktiven Gruppe in ω-Stellung beinur geringem Verlust an biologischer Aktivität an polymere Träger binden lassen. Die erfindungsgemäßen Verbindungen sind zur Inhibierung der Thrombozytenaggregation, zur Blutdrucksenkung über eine Vasodilation, zur Hemmung der Magensäuresekretion sowie nach chemischer Verknüpfung mit Proteinen zur Darstellung von Antikörpern zu Carbacyclinen geeignet.

Die Erfindung betrifft Carbacyclinderivate der Formel I

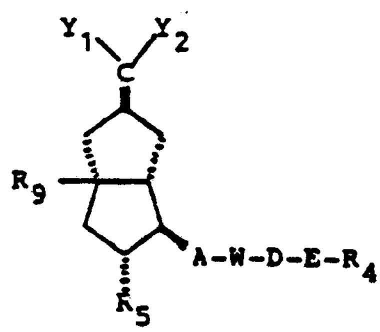 (I),

worin

$Y_1$ den Rest $-CH_2$-X-$(CH_2)_n$-$R_1$ oder den Rest

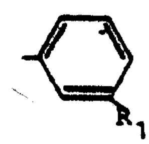

n 1 oder 3,

$R_1$ den Rest

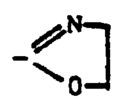 , den Rest 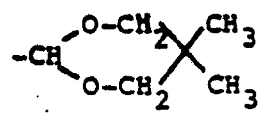 ,

$-COCH_3$, $COOR_2$, wobei $R_2$ Wasserstoff oder gegebenenfalls durch Halogen, Phenyl, $C_1$-$C_4$-Alkoxy oder $C_1$-$C_4$-Dialkyl-amino substituiertes Alkyl mit 1-10 C-Atomen, Cycloalkyl, Aryl oder einen heterocyclischen Rest bedeuten kann, oder den Rest $CONHR_3$ mit $R_3$ in der Bedeutung Wasserstoff oder eines Alkanoyl- oder Alkansulfonylrestes mit je 1-10 C-Atomen darstellt,

$R_9$ den Rest $-Z-(CH_2)_m-R_6$

m = 2-10,

Z eine $-C{\equiv}C-$, eine cis-CH=CH— oder eine trans-CH=CH— Gruppe bedeutet,

$R_6$ $N_3$, Cl, Br, J oder $COOR_2$, bedeutet,

$R_2$ Wasserstoff oder eine Methyl- oder Ethylgruppe,

X ein Sauerstoffatom oder eine Methylengruppe,

$Y_2$ Wasserstoff oder Fluor,

A eine $-CH_2-CH_2-$, trans-CH=CH— oder $-C{\equiv}C$-Gruppe,

W eine freie oder funktionell abgewandelte Hydroxymethylengruppe oder eine freie oder funktionell abgewandelte

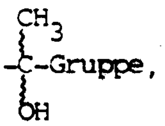

wobei die OH-Gruppe α- oder β-ständig sein kann,

D die Gruppe

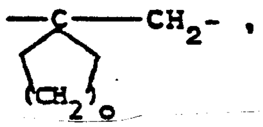

eine geradkettige, gesättigte Alkylengruppe mit 1-5 C-Atomen, eine verzweigte gesättigte oder eine geradkettige oder verzweigte ungesättigte Alkylengruppe mit 2-5 C-Atomen, die gegebenenfalls durch Fluoratome substituiert sein können,

o 1,2 oder 3 bedeutet,

E eine Direktbindung, eine —C≡C-Gruppe oder eine —CH=CR$_7$-Gruppe darstellt, wobei R$_7$ ein Wasserstoffatom, eine Alkylgruppe mit 1-5 C-Atomen oder Halogen bedeuten,

R$_4$ eine Alkylgruppe mit 1-10 C-Atomen, eine Cycloalkylgruppe mit 3-10 C-Atomen oder eine gegebenenfalls substituierte Arylgruppe mit 6-10 C-Atomen oder eine heterocyclische Gruppe,

R$_5$ eine freie oder funktionell abgewandelte Hydroxygruppe, und, falls R$_2$ die Bedeutung eines Wasserstoffatoms hat, deren Salze mit physiologisch verträglichen Basen bedeuten und deren Cyclodextrinclathrate.

Als Alkylgruppen R$_2$ sind gerad- oder verzweigtkettige Alkylgruppen mit 1-10 C-Atomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.

Die Alkylgruppen R$_2$ können gegebenenfalls 1 bis mehrfach substituiert sein durch Halogenatome, C$_1$-C$_4$-Alkoxygruppen, Phenyl und Di-C$_1$-C$_4$-alkylamine. Bevorzugt sind solche Alkylgruppen, die einfach substituiert sind.

Als Substituenten seien beispielsweise genannt Fluor-, Chlor- oder Bromatome, Phenyl, Dimethylamino, Diethylamino, Methoxy, Ethoxy. Als bevorzugte Alkylgrupen R$_2$ sind solche mit 1-4 C-Atomen, wie z.B. Methyl, Ethyl, Propyl, Dimethylaminopropyl, Isobutyl, Butyl zu nennen.

Als Arylgruppen R$_2$ kommen sowohl substituierte wie auch unsubstituierte Arylgruppen in Betracht, wie beispielsweise Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, Hydroxy- oder Alkoxygruppe mit 1-4 C-Atomen.

Bevorzugt sind die Substituenten in 3- und 4-Stellung am Phenylring, zum Beispiel durch Fluor, Chlor, Alkoxy oder Trifluormethyl oder in 4-Stellung durch Hydroxy.

Die Cycloalkylgruppe R$_2$ kann im Ring 4-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methylcyclohexyl und Adamantyl.

Als heterocyclische Gruppen R$_2$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 3-Furyl, 2-Thienyl, 3-Thienyl, 2-Pyridyl, 3-Pyridyl, 4-Pyridyl u.a.

Als Säurerest R$_3$ kommen physiologisch verträgliche Säurereste in Frage. Bevorzugte Säuren sind organische Carbonsäuren und Sulfonsäuren mit 1-10 C-Atomen, die der aliphatischen, cycloaliphatischen, aromatischen, aromatisch-aliphatischen und heterocyclischen Reihe angehören. Diese Säuren können gesättigt, ungesättigt und/oder mehrbasisch und/oder in üblicher Weise substituiert sein. Als Beispiele für die Substituenten seien C$_1$-C$_4$-Alkyl, Hydroxy-, C$_1$-C$_4$-Alkoxy-, Oxo- oder Aminogruppen oder Halogenatome (F, Cl, Br) erwähnt.

Beispielsweise seien folgende Carbonsäuren genannt : Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure. Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste werden solche mit bis zu 4 Kohlenstoffatomen betrachtet. Als Sulfonsäuren kommen beispielsweise Methansulfonsäure, Ethansulfonsäure, Isopropansulfonsäure, Cyclohexansulfonsäure, N,N-Dimethylaminosulfonsäure, N,N-Diethylaminosulfonsäure, N,N-Bis-(β-chlorethyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage, wobei Sulfonsäuren mit bis zu 4 C-Atomen besonders bevorzugt sind.

Die Hydroxygruppen $R_5$ und in W können funktionell abgewandelt sein, beispielsweise durch Veretherung oder Veresterung, wobei die freien oder abgewandelten Hydroxygruppen in W α- oder β-ständig sein können, wobei freie Hydroxygruppen bevorzugt sind.

Als Ether- und Acylreste kommen die dem Fachmann bekannten Reste in Betracht. Bevorzugt sind leicht abspaltbare Etherreste wie beispielsweise der Tetrahydropyranyl-, Tetrahydrofuranyl-, α-Ethoxyethyl-, Trimethylsilyl-, Dimethyl-tert.-butyl-silyl- und Tri-p-benzyl-silylrest. Als Acylreste kommen die gleichen wie für $R_3$ genannt in Frage ; namentlich genannt seien beispielsweise Acetyl, Propionyl, Butyryl, Benzoyl.

Als Alkylgruppe $R_4$ kommen gerad- und verzweigtkettige, gesättigte und ungesättigte Alkylreste, vorzugsweise gesättigte, mit 1-10, insbesondere 1-4 C-Atomen, in Frage.

Beispielsweise genannt seien Methyl-, Ethyl-, Propyl-, Butyl-, Isobutyl-, tert.-Butyl-, Pentyl-, Hexyl-, Heptyl-, Octyl-, Butenyl-, Isobutenyl-, Propenyl-, Pentenyl-, Hexenyl-.

Die Cycloalkylgruppe $R_4$ kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl, Methyl-cyclohexyl und Adamantyl.

Als substituierte bzw. unsubstituierte Arylgruppen $R_4$ kommen beispielsweise in Betracht : Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, $C_1$-$C_4$-Alkoxy- oder Hydroxygruppe. Bevorzugt ist die Substitution in 3- und 4-Stellung am Phenylring zum Beispiel durch Fluor, Chlor, $C_1$-$C_4$-Alkoxy oder Trifluormethyl oder in 4-Stellung durch Hydroxy.

Als heterocyclische Gruppen $R_4$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, 3-Furyl, 3-Thienyl u.a.

Als Alkylengruppe D kommen geradkettige oder verzweigtkettige, gesättigte und ungesättigte Alkylenreste, vorzugsweise gesättigte mit bis zu 5 C-Atomen, in Frage, die gegebenenfalls durch Fluoratome, 1,2-Methylen, 1,1-Trimethylen, 1,1-Tetramethylen oder 1,1-Pentamethylen substituiert sein können. Beispielsweise seien genannt : Methylen, Fluormethylen, Ethylen, 1,2-Propylen, Ethylethylen, Trimethylen, Tetramethylen, Pentamethylen, 1-Methyltetramethylen, 1-Methyl-trimethylen, 1,1-Trimethylenethylen, 1,1-Tetramethylenethylen.

Besonders bevorzugte Verbindungen dieser Erfindung sind solche mit E als $—C{\equiv}C$ oder $—CH{=}CR_7$, worin $R_7$ eine Alkylgruppe mit 1-5 C-Atomen darstellt.

Als Alkylgruppe $R_7$ mit 1-5 C-Atomen kommen die für die Alkylgruppe $R_4$ bereits genannten Gruppe in Frage.

Mit $R_7$ als Halogen sind Fluor, Chlor und Brom gemeint.

Für $R_9$ als $—Z\text{-}(CH_2)_m\text{-}R_5$ kommen Alkin- oder Alkylengruppen mit 2-20 C-Atomen in Betracht, wobei m = 2-20 ist, wie z.B. $—C{\equiv}C(CH_2)_m\text{-}N_3$, $—C{\equiv}C\text{-}(CH_2)_m\text{-}CO_2H$, $—C{\equiv}C\text{-}(CH_2)_m\text{-}Br$.

Zur Salzbildung mit den freien Säuren ($R_2$=H) sind anorganische und organische Basen geeignet, wie sei dem Fachmann zur Bildung physiologisch verträglicher Salze bekannt sind. Beispielsweise seien genannt : Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Die Erfindung betrifft ferner Verfahren zur Herstellung der erfindungsgemäßen Carbacycline der allgemeinen Formel I, die dadurch gekennzeichnet sind, daß man a) eine Verbindung der Formel IV

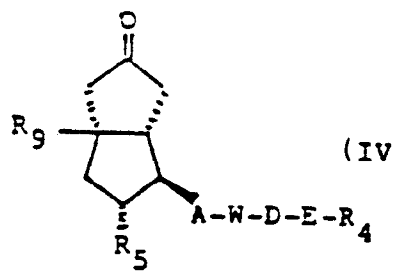

(IV),

worin A, W, D, E, $R_4$, $R_5$ und $R_9$ die bereits angegebenen Bedeutungen haben, und mit einem Wittigreagenz der allgemeinen Formeln V und VI oder einem Dianion der Formel VII

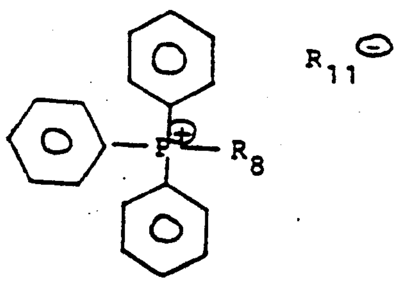

(V)

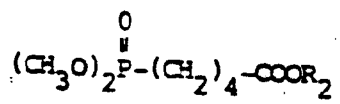

(VI)

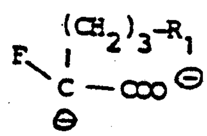

(VII),

worin

$R_2$ die genannte Bedeutung und $R_8$ die Reste —$CH_2$-$CH_2$-X-$(CH_2)_n$-$R_1$ oder

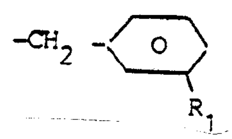

mit den bereits erwähnten Bedeutungen für X, n und $R_1$ und $R_{11}$ Brom oder Chlor bedeuten, in Gegenwart von K -tert.-Butylat umsetzt oder b) eine Verbindung der Formel VIII, die man aus dem entsprechenden 4-Ester durch DIBAH-Reduktion erhält,

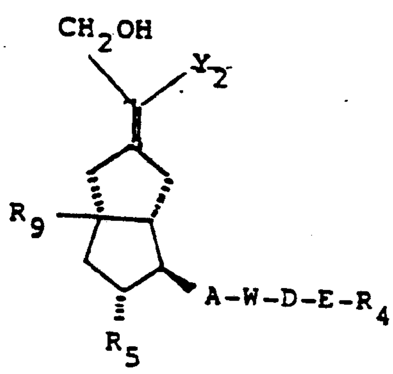

(VIII),

worin A, W, D, E, $R_4$, $R_5$, $R_9$ und $Y_2$ die bereits angegebenen Bedeutungen haben, gegebenenfalls nach Schutz anwesender freier Hydroxy- oder Aminogruppen mit einem Halogenalkansäurederivat der Formel IX

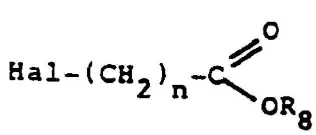

(IX),

wobei n 1 oder 3, Hal ein Chlor- oder Bromatom und $R_8$ einen Alkylrest mit 1-4 C-Atomen oder ein Alkalimetall bedeuten, in Gegenwart einer Base verethert und gegebenenfalls anschließend in beliebiger Reihenfolge Isomere trennt und/oder geschützte Hydroxygruppen freisetzt und/oder freie Hydroxygruppen verestert, verethert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

Die Umsetzung der Verbindung der allgemeinen Formel VIII mit einem Halogenalkansäurederivat der allgemeinen Formel IX wird bei Temperaturen von 0°C bis 100°C, vorzugsweise 10°C bis 80°C, in einem aprotischen Lösungsmittel oder Lösungsmittelgemisch, beispielsweise Dimethylsulfoxid, Dimethylformamid, Tetrahydrofuran usw., vorgenommen. Als Basen kommen die dem Fachmann für Veretherungen bekannten Basen in Frage, beispielsweise Natriumhydrid, Kalium-tert.-butylat, Butyllithium, usw.

Die Ausgangsverbindungen der Formel IV erhält man durch Umsetzung von Verbindungen der Formel II

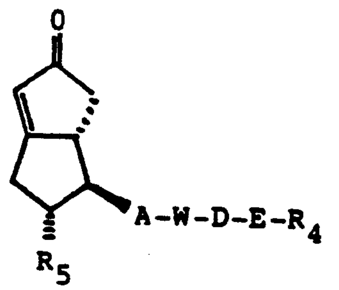

(II),

worin

$R_4$, $R_5$, A, W, D, E, $Z_1$, $Z_2$, m, o, p, q die angegebenen Bedeutungen haben, mit Metall-Reagenzien der Formel III,

$$R_{10}\text{-}(CH_2)_m\text{-}Z\text{-}R_{12} \quad \text{(III)},$$

worin $R_{12}$ MgBr, MgCl, MgJ oder Al $(Alkyl)_2$ mit Alkyl als gerad- oder verzweigtkettigem Alkylrest mit bis zu 6 C-Atomen bedeutet, durch 1,4-Addition und anschließende Einführung der oberen Seitenkette durch Wittig-Reaktion der 5-Ring-Carbonylgruppe und Umwandlung der Gruppe $R_{10}$ in die Gruppe $R_6$. Für die Gruppe $R_{10}$ kommen geschützte Aminogruppen wie z.B. durch 1,1,4,4-Tetramethyl-1,4-dichlordisilylethan oder durch Phthalsäureanhydrid bzw. durch andere typische Aminoschutzgruppen geschützte Aminogruppen sowie durch tert.-Butyldiphenylsilyl- oder THP-Gruppen geschützte Hydroxylgruppen sowie durch Umwandlung in Orthoester oder Oxazolin -geschützte Carboxylgruppen in Betracht.

Nach Abspaltung der Schutzgruppen vom Stickstoff bzw. nach chemischer Umwandlung der Hydroxy- oder Amidgruppen in Aminogruppen erhält man die gewünschten Substituenten $R_6{=}NH_2$. Für diese Umwandlung einer Hydroxygruppe in eine Aminogruppe kann man die Mitsunobu-Reaktion (s. Synthesis 1, 1981) bzw. die Reduktion des Azids zum Amin verwenden.

Die Verseifung der Carbacyclinester wird nach den dem Fachmann bekannten Methoden durchgeführt, beispielsweise mit basischen Katalysatoren.

Die Einführung der Estergruppe $COOR_2$ für $R_1$, bei welcher $R_2$ eine Alkylgruppe mit 1-10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diethylether mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389-394 (1954)].

Die Einführung der Estergruppe $COOR_2$ für $R_1$, bei welcher $R_2$ eine substituierte oder unsubstituierte Arylgruppe darstellt, erfolgt nach den dem Fachmann bekannten Methoden. Beispielsweise werden die Carboxyverbindungen mit den entsprechenden Arylhydroxyverbindungen mit Dicyclohexylcarbodiimid in Gegenwart einer geeigneten Base, beispielsweise Pyridin oder Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform, in Frage. Die Reaktion wird bei Temperaturen zwischen – 30°C und + 50°C, vorzugsweise bei + 10°C, durchgeführt.

Die Carbacyclin-Derivate der allgemeinen Formel I mit $R_1$ in der Bedeutung einer Carboxygruppe können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, zum Beispiel Alkohol doer Aceton, das feste anorganische Salz.

Die Herstellung der Aminsalze erfolgt in üblicher Weise. Dazu wird die PG-Säure zum Beispiel in einem geeigneten Lösungsmittel, wie Ethanol, Aceton, Diethylether oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Etherschutzgruppen wird beispielsweise mit Dihydropyran in Methylenchlorid oder Chloroform unter Verwendung eines sauren Kondensationsmittels, wie zum Beispiele p-Toluolsulfonsäure, umgesetzt. Das Dihydropyran wird im Überschuß angewandt, vorzugsweise in der 4- bis 10-fachen Menge des theoretischen Bedarfs. Die Umsetzung ist normalerweise bei 0°C-30°C nach 15-30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Etherschutzgruppen in einer wäßrigen Lösung einer organischen Säure, wie zum Beispiel Essigsäure, Propionsäure u.a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Ethanol, und Ether, wie Dimethoxyethan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Abspaltung der Silyletherschutzgruppen erfolgt beispielsweise mit Tetrabutylammoniumfluorid. Als Lösungsmittel sind beispielsweise geeignet Tetrahydrofuran, Diethylether, Dioxan, Methylenchlorid usw. Die Abspaltung wird vorzugsweise bei Temperaturen zwischen 0°C und 80°C durchgeführt.

Die Verseifung der Acylgruppen erfolgt beispielsweise mit Alkali- oder Erdalkali-carbonaten oder -hydroxyden in einem Alkohol oder der wäßrigen Lösung eines Alkohols. Als Alkohole kommen aliphatische Alkohole in Betracht, wie zum Beispiel Methanol, Ethanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei – 10°C bis 70°C, vorzugsweise bei 25°C.

Die Einführung der Amidgruppe $CONHR_3$ für $R_1$ erfolgt nach den dem Fachmann bekannten Methoden. Die Carbonsäure der allgemeinen Formel I ($R_2$=H) werden zunächst in Gegenwart eines tertiären Amins, wie beispielsweise Triethylamin, mit Chlorameisensäureisobutylester in das gemischte Anhydrid überführt. Die Umsetzung das gemischten Anhydrids mit dem Alkalisalz des entsprechenden Amids oder mit Ammoniak ($R_3$=H) erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen – 30°C und + 60°C, vorzugsweise bei 0°C bis 30°C.

Eine weitere Möglichkeit für die Einführung der Amidgruppe $CONHR_3$ für $R_1$ besteht in der Umsetzung einer 1-Carbonsäure der allgemeinen Formel I ($R_2$=H), in der freie Hydroxygruppen intermediär geschützt sind, mit Verbindungen der allgemeinen Formel X

$$O{=}C{=}N{-}R_3 \quad (X),$$

worin $R_3$ die obenangegebene Bedeutung hat.

Die Umsetzung der Verbindung der allgemeinen Formel I ($R_2$=COOH) mit einem Isocyanat der allgemeinen Formel VIII erfolgt gegebenenfalls unter Zusatz eines tertiären Amins, wie z.B. Triethylamin oder Pyridin. Die Umsetzung kann ohne Lösungsmittel oder in einem inerten Lösungsmittel, vorzugsweise Acetonitril, Tetrahydrofurna, Aceton, Dimethylacetamid, Methylenchlorid, Diethylether, Toluol, bei Temperaturen zwischen –80°C bis 100°C, vorzugsweise bei 0°C bis 30°C, vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxygruppen im Prostanrest enthalten, geht man zweckmäßigerweise von Ausgangsprodukten aus, in denen diese durch vorzugsweise leicht abspaltbare Ether- oder Acylreste intermediär geschützt sind.

Alle übrigen Verbindungen der Formel I können nach Verfahren hergestellt werden, wie sie in den Offenlegungsschriften DE-OS 2845770, 3237200, 3322893 und 3405181 beschrieben werden.

Die Carbacycline der Formel I, in denen $R_9$ den Rest $R_6$-$(CH_2)_m$-Z — mit $R_6$ als $N_3$, Cl, Br, J, $COOR_2$-Gruppe bedeutet, lassen sich sehr gut ohne größeren Verlust an biologischer Aktivität an polymere Träger binden. Durch die neuen Carbacycline wird verhindert, daß sich an der Oberfläche dieser polymeren Träger wie z.B. Gefäßprothesen oder künstlichen Herzklappen Thrombozytenaggregate bilden. Nach chemischer Verknüpfung mit Proteinen eignen sich die Verbindungen der Formel I zur Darstellung von Antikörpern zu Prostacyclinen der allgemeinen Formel I.

Die Verbindungen dieser Erfindung eignene sich ferner zur Therapie von Erkrankungen des cardiovaskulären Systems, des Magens, des Pankreas, der Leber und der Niere. Sie wirken blutdrucksenkend und bronchodilatorisch. Sie sind weiterhin geeignet zur Hemmung der Thrombozyten-Aggregation. Folglich stellen die neuen Carbacyclin-Derivate der Formel I wertvolle pharmazeutische Wirkstoffe dar. Darüber hinaus weisen sie bei ähnlichem Wirkungsspektrum, verglichen mit entsprechenden Prostaglandinen und Prostacyclinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Carbacycline zeigt sich bei der Untersuchung an glattmuskulären Organen, wie z.B. am Meerschweinchenileum oder an der isolierten Kaninchentrachea, wo eine wesentlich geringere stimulation zu beobachten ist als bei der Applikation natürlicher Prostaglandine vom E-, A- oder F-Typ.

Die neuen Carbacyclin-Analoga besitzen die für Prostacycline typischen Eigenschaften, wie z.B. Senkung das peripheren arteriellen und koronaren vaskulären Widerstandes, Inhibierung der Thrombozytenaggregation und Auflösung von Plättchenthromben, myocardiale Zytoprotektion, Senkung des systemischen Blutdruckes ohne zugleich Schlagvolumen und koronare Durchblutung zu senken ; Behandlung von Schlaganfall, Prophylaxe und Therapie koronarer Herzerkrankungen, koronarer Thrombose, des Therapie koronarer Herzerkrankungen, koronarer Thrombose, des Herzinfarkts, peripherer Arterienerkrankungen, Arteriosklerose und Thrombose, Prophylaxe und Therapie ischaemischer Attacken des ZNS-Systems, Therapie des Schocks, Inhibierung der Bronchokonstriktion, Inhibierung der Magensäuresekretion und Zytoprotektion der Magen- und Darmschleimhaut, Zytoprotektion in der Leber, im Pankreas und in der Niere, antiallergische Eigenschaften, Senkung des pulmonalen vaskulären Widerstandes und des pulmonalen Blutdruckes, Förderung der Nierendurchblutung, Anwendung anstelle von Heparin oder als Adjuvans bei der Dialyse oder Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung, Behandlung von Asthma, etc. Außerdem besitzen die neuen Carbacyclinanaloga antoproliferative Eigenschaften. Die neuen Carbacycline können außerdem in Kombination z.B. mit β-Blockern oder Diuretika Verwendung finden.

Die neuen Carbacycline zeichnen sich außerdem noch durch Unterdrückung von Abstoßungsreaktionen und durch ihre antimetastatische Wirkung aus. Durch sie wird der Ductus Botalli (vor Operationen) offengehalten. Sie eignen sich ferner zur Durchfallbehandlung und zur Verbesserung des Stuhlganges.

Die Dosis der Verbindungen ist 1-1500 µg/kg/Tag, wenn sie am menschlichen Patienten verabreicht werden. Die Einheitsdosis für den pharmazeutischen akzeptablen Träger beträgt 0,01-100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z.B. zur Herstellung von Blutdrucksenkern dienen.

Der Einheitsdosisbereich für die Ampulle ist 0,1-0,5 mg, für die Tablette 01,-1 mg.

Die neuen Carbacycline binden bei nur geringem Verlust an biologischer Aktivität an polymere Träger. So hemmt die Verbindung aus Beispiel 1 die Thrombozytenaggregation in einer Konzentration von $IC_{50} = 1{,}7 \cdot 16^{-8}$ M(0,14 × Iloprost). Die Verbindung aus Beispiel 1 eignet sich außerdem zur Bestimmung potentieller Prosta- und Carbacycline am Prostacyclin-Rezeptor.

Beispiel 1

5(Z)-{1(S,β)-[5-Azido-1-pentinyl]-6β-[(E)-3α-hydroxy-4-methyl-oct-6-in-1-enyl]-7α-hydroxy-bicyclo [3.3.0] octan-3-yliden}-pentansäure

1a

Die auf – 40°C gekühlte Lösung von 4.20 g (13,0 mmol) 1-Pentin-5-yl-tert.-butyldiphenylsilylether in 25 ml wasserfreiem Diethylether versetzte man mit 8,4 ml einer 1,54 molaren Lösung von n-Buli in Hexan und nach 30 minütigem Rühren mit 13 ml einer 1 molaren Lösung von Diethylaluminiumchlorid in Toluol. Nach beendeter Zugabe ließ man auf 0-3°C erwärmen und weitere 45 Minuten reagieren. Die entstandene Emulsion gab man zu einem gemisch aus 352 mg (1,37 mmol) Nickelacetylacetonat, das man in 25 ml wasserfreiem Diethylether aufgeschlammt und mit 1,3 ml einer 1 molaren Lösung aus Diisobutylaluminiumhydrid in Toluol versetzt hatte, kühlte auf – 10°C und tropfte zügig die Lösung von 2,40 g (5,42 mmol) 7-[(R,α)-Tetrahydropyran-2-yl-oxy)-6β-[(E)-4-methyl-3α-(tetrahydropyran-2-yl-oxy)-oct-6-in-1-enyl]-bicyclo [3.3.0] oct-1-en-3-on in 25 ml wasserfreiem Diethylether zu. Man ließ noch 1,5 h bei – 10°C bis – 5°C reagieren, quenchte durch Eingießen in ein gut gerührtes Gemisch aus ca. 300 ml fein gestoßenem Eis und 100 ml 0,5 n HCl, extrahierte mehrfach mit insgesamt 400 ml Diethylether, wusch die vereinigten organischen Extrakte zunächst mit Wasser, dann mit ges. NaCl-Lösung bis zur Neutralreaktion, trocknete über Magnesiumsulfat und entfernte das Lösungsmittel im Wasserstrahlvakuum.

Das braune Rohöl chromatographierte man unter Druck an einer Kieselgelsäule unter Verwendung eines n-Hexan/Essigester — Gradientensystems.

Man isolierte 2,20 g (2,88 mmol, 53%) noch leicht verunreinigtes 1(S,β)-[5-tert.-Butyldiphenylsilyloxy-1-pentinyl]-7α-tetrahydropyran-2-yl-oxy-6β-[(E)-3α-(tetrahydropyran-2-yl-oxy)-4-methyl-oct-6-in-1-enyl]-bicyclo [3.3.0] oct-1-en-3-on.

IR (Film) : 3065, 3040, 2820-2980, 1740, 1590, 1425, 1200, 1108, 1075, 1020, 970, 868, 820, 740 und 700 $cm^{-1}$.

1b

Die Emulsion aus 2,46 g 55%iger NaH-Dispersion in Weissöl versetzte man mit 160 ml wasserfreiem DMSO, ließ 1 h bei 23°C reagieren und versetzte sodann portionsweise innerhalb 5 Minuten mit insgesamt 13,1 g (29,6 mmol) Carboxybutyltriphenylphosphoniumbromid. Anschließend tropfte man innerhalb 45 Minuten die Lösung des unter 1a dargestellten Ketones in 50 ml DMSO zu und ließ 28 h bei 50 bis 55°C reagieren. Man goß auf ein Gemisch aus ca. 200 ml Eis und 200 ml Wasser, stellte mit gesättigter Citronensäure auf pH 6 ein und extrahierte mehrfach mit insgesamt 400 ml Diethylether. Die weitere Aufarbeitung wurde wie unter 1a beschrieben durchgeführt. Das erhaltene Rohprodukt chromatographierte man unter Druck an einer Kieselgelsäule unter Verwendung eines Dichlormethan/Ethanol-Gradientensystems. Man isolierte 1,64 g (1,93 mmol, 67%) 5(E/Z)-{1(S,β-[5-tert.-Butyldiphenyl-silyloxy-1-pentinyl]-6β-[(E)-3α-(tetrahydropyran-2-yloxy)-4-methyl-oct-6-in-1-enyl]-7α-(tetrahydropyran-2-yloxy)-bicyclo [3.3.0] octan-3-yliden}-pentansäure.

IR (Film) : 2600-3600, 3070, 3050, 2820-2980, 1710, 1590, 1428, 1260, 1200, 1110, 1020, 970, 870, 820, 738 und 704 $cm^{-1}$.

1c

780 mg (0,92 mmol) der unter 1b isolierten Carbonsäuren veresterte man mit einer etherischen Lösung von Diazomethan und isolierte nach chromatographischer Reinigung an einer Kieselgelsäule unter Verwendung eines Laufmittelgemisches aus n-Hexan/Essigester 649 mg (0,75 mmol, 82%) sauberen 5(E/Z)-{1(S,β)-[5-tert.-Butyldiphenylsilyloxy-1-pentinyl]-6β-[(E)-3α-(tetrahydropyran-2-yl-oxy)-4-methyl-oct-6-in-1-enyl]-7α-( tetrahydropyran-2-yl-oxy)-bicyclo [3.3.0] octan-3-yliden}-pentansäure-methylester.

IR (Film) : 3070, 3040, 2820-2980, 1738, 1590, 1428, 1200, 1110, 1075, 1020, 972, 870, 820, 740 und 703 $cm^{-1}$.

1d

146 mg (169 μmol) des unter 1c beschriebenen Methylesters löste man in 30 ml wasserfreiem Tetrahydrofuran, versetzte mit 0,3 ml einer 1 molaren Tetrabutylammoniumfluoridlösung in Tetrahydrofuran und rührte 18 h bei 23°C. Man quenchte durch Zugabe von 30 ml einer 10%igen wässrigen Ammoniumchlorid-Lösung und extrahierte mehrfach mit insgesamt 80 ml Diethylether. Die organischen Extrakte wusch man mit ges. NaCl-Lösung, trocknete über Magnesiumsulfat und reinigte das nach Abzug des Lösungsmittels im Wasserstrahlvakuum erhaltene Rohprodukt durch Chromatographie an 6 DC-Platten mit einem 1 : 1-Gemisch aus n-Hexan/Essigester. Man isolierte nach Elution mit Diethylether 91 mg (146 μmol, 86%) 5(E/Z)-{1(S,β)-[5-Hydroxy-1-pentinyl]-6β-[(E)-3α-(tetrahydropyran-2-yl-oxy)-4-methyl-oct-6-in-1-enyl]-7α-(tetrahydropyran-2-yl -oxy)-bicyclo [3.3.0] octan-3-yliden-} pentansäure-methylester.

IR (Film) : 3200-3650, 2820-2980, 1740, 1430, 1200, 1075, 1020, 970, 902, 867 und 815 $cm^{-1}$.

1e

Den unter 1d erhaltenen Alkohol löste man in 11 ml wasserfreiem Acetonitril und versetzte nacheinander mit 484 mg (1,46 mmol) Tetrabrommethan, 117 μl (1,45 mmol) Pyridin, 382 mg (1,46 mmol) Triphenylphosphin, 335 μl (1,45 mmol) Kollidin und ließ 1 h bei 23°C reagieren. Man versetzte mit 2 ml Wasser, 1 ml 0,5 n Salzsäure, 2 Tropfen einer wässrigen 10%igen $Na_2S_2O_3$-Lösung und extrahierte mehrfach mit 100 ml Diethylether. Nach üblicher Aufarbeitung reinigte man das Rohprodukt durch Chromatographie an 9 DC-Platten unter Verwendung eines 4 : 1-Gemisches aus n-Hexan/Essigester. Nach Elution mit Diethylether isolierte man 93 mg (136 μmol, 93%) 5(E/Z)-{1(S,β)-[5-Brom-1-pentinyl]-6β-[(E)-3α-(tetrahydropyran-2-yl-oxy)-4-methyl-oct-6-in-1-enyl]-7α-(tetrahydropyran-2-yl-oxy)-bicyclo [3.3.0] octan-3-yliden}-pentansäure-methylester.

IR (Film) : 2820-2980, 1740, 1452, 1375, 1200, 1160, 1115, 1075, 1020, 975, 906, 870 und 815 $cm^{-1}$.

1f

Die unter 1e erhaltenen Bromide löste man in 9 ml wasserfreiem Dimethylformamid, versetzte mit 38 mg 585 μmol) Natriumazid und ließ 3,5 h bei 60°C reagieren. Man versetzte mit 60 ml Wasser, extrahierte mehrfach mit insgesamt 100 ml Diethylether, wusch mit ges. NaCl, trocknete über Magnesiumsulfat und zog das Lösungsmittel im Wasserstrahlvakuum ab. Isoliert wurden 141 mg roher 5(E/Z)-{1(S,β)-[5-Azido-1-pentinyl]-6β-[(E)-3α-(tetrahydropyran-2-yl-oxy)-4-methyl-oct-6-in-1-enyl]-7α-(tetrahydropyran-2-yl-oxy)-bicyclo [3.3.0] octan-3-yliden}-pentansäure-methylester, der ohne Reinigung weiter umgesetzt wurde.

1g

Die unter 1f erhaltenen Azide versetzte man mit 18 ml eines 65 : 35 : 10-Gemisches aus Eisessig/Wasser/Tetrahydrofuran und rührte 23 h bei 23°C. Man engte im Wasserstrahlvakuum bei 40°C ein und entfernte noch enthaltene Essigsäure mittels azeotroper Destillation durch wiederholte Zugabe von Toluol. Das so erhaltene Rohprodukt reinigte man durch Chromatographie an 9 DC-Platten unter Verwendung eines 95 : 5-Gemisches von Dichlormethan/Ethanol als Laufmittel und Diethylether als Elutionsmittel. Man isolierte 51 mg (106 μmol, 78% bezogen auf Edukt von 1f) (5(E/Z)-{1(S,β)-[5-Azido-1-pentinyl]-6β-[(E)-3α-hydroxy-4-methyloct-6-in-1-enyl]-7α-hydroxy-bicyclo [3.3.0] octan-3-yliden}-pentansäuremethylester.

IR (Film) : 3200-3600, 2820-2980, 2100, 1734, 1435, 1200, 1085, 1030, 1020 und 970 $cm^1$.

1h

50 mg der unter 1g isolierten Methylester löste man in 6 ml Methanol, versetzte mit 4 ml einer 5%igen wässrigen Lithiumhydroxidlösung, stellte nach 4 h Rühren bei 23°C durch Zugabe einer gesättigten Citronensäure auf pH 3.4 ein, extrahierte mehrfach mit Trichlormethan und trocknete die vereinigten organischen Extrakte über Magnesiumsulfat. Das nach Abzug des Lösungsmittels im Wasserstrahlvakuum erhaltene Rohprodukt trennte man durch Chromatographie an 4 Kieselgelplatten unter Verwendung eines 9 : 1-Gemisches aus Dichlormethan/Ethanol, zweimaliger Entwicklung und Elution mit Trichlormethan/Isopropanol auf. Nach Abzug des Lösungsmittels isolierte man 17 mg (36 μmol, 35%) 5(E)-{1(S,β)-[5-Azido-1-pentinyl]-6β-[(E)-3α-hydroxy-4-methyl-oct-6-in-1-enyl]-7α-hydroxy-bicyclo [3.3.0] octan-3-yliden}-pentansäure sowie 19 mg (41 μmol, 39%)

der Titelverbindung.

IR (Film) : 2450-3700, 2820-2980, 2100, 1710, 1570, 1430, 1410, 1295, 1280, 1095, 1075, 1018 und 970 cm1.

Beispiel 2

5(E/Z)-{1(S,β)-[4-Carboxy-1-butinyl]-6β-[(E)-3α-hydroxy-4-methyl-oct-6-in-1-enyl]-7α-hydroxy-bicyclo [3.3.0] octan-3-yliden}-pentansäuremethylester.

2a

193 mg (309 μmol) 5(E/Z)-{1(S,β)-[5-Hydroxy-1-pentinyl]-6β-[(E)-3α-(tetrahydropyran-2-yl-oxy)-4-methyl-oct-6-in-1-enyl]-7α-(tetrahydropyran-2-yl-oxy)-bicyclo [3.3.0] octan-3-yliden}-pentansäuremethylester löste man in 25 ml Aceton, kühlte auf – 30°C, versetzte mit 600 μl Jones-Reagenz und ließ 3 h bei – 30°C bis – 20°C reagieren. Überschüssiges Oxidationsmittel entfernte man durch Zugabe von 2 ml Isopropanol, ließ auf 20°C erwärmen, vedünnte mit Wasser und extrahierte mit Dichlormethan. Die vereinigten organischen Extrakte wusch man zunächst mit Wasser, dann mit ges. NaCl-Lösung bis zur Neutralreaktion, trocknete über Magnesiumsulfat und reinigte das nach Lösungsmittelabzug erhaltene Rohöl durch Chromatographie an 10 Kieselgelplatten. Als Laufmittel diente ein 2 : 8-Gemisch aus n-Hexan/Essigester, als Elutionsmittel ein Gemisch aus Trichlormethan/Isopropanol. Man isolierte neben 30 mg Ausgangsmaterial 157 ml (246 μmol, 80%) 5(E/Z)-{1(S,β)-[4-Carboxy-1-butinyl]-6β-[(E)-3α-(tetrahydropyran-2-yl-oxy)-4-methyl-oct-6-in-1-enyl]-7α-(tetrahydro pyran-2-yl-oxy)-bicyclo [3.3.0] octan-3-yliden}-pentansäure-methylester.

IR (Film) : 2500-3600, 2820-2980, 1732, 1710, 1435, 1265, 1200, 1130, 1020, 974, 904, 867 und 812 $cm^{-1}$.

2b

Von dem in 2a erhaltenen Produkt wurden in Analogie zu Beispiel 1g die Tetrahydropyranylether gespalten. Nach chromatographischer Reinigung auf 7 Kieselgelplatten mit einem 95 : 5-Gemisch aus Dichlormethan/Methanol, zweimaliger Entwicklung und Elution mit einem 8 : 2-Gemisch aus Dichlormethan/i-Propanol isolierte man neben 47 mg Ausgangsmaterial 69 mg (146 μmol, 60%) der Titelverbindung.

IR (Film) : 3100-3500, 2500-3650, 2820-2980, 1735, 1710, 1435, 1260, 1200, 1074, 1020, 970 und 902 $cm^{-1}$

Beispiel 3

5(E/Z)-{(1S,β)-[4-Carboxy-1-butinyl]-6β-[(E)-3α-hydroxy-4-methyl-oct-6-in-1-enyl]-7α-hydroxy-bicyclo [3.3.0] octan-3-yliden}-pentansäure.

14 mg (30 μmol) der nach Beispiel 2 erhaltenen Titelverbindung setzte man in Analogie zu Beispiel 1 h um. Nach analoger Aufarbeitung isolierte man 13 mg (29 μmol, 95%) der Titelverbindung.

IR (Film) : 2500-3650, 2820-2980, 1712, 1570, 1435, 1075, 1020, 970 $cm^{-1}$.

## Patentansprüche

1. Carbacyclinderivate der Formel I

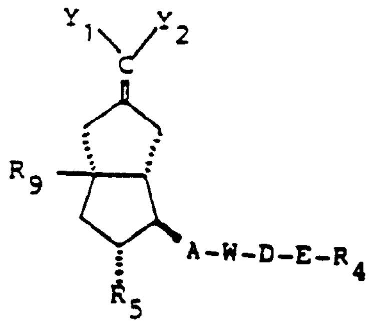

(I),

worin

$Y_1$ den Rest $—CH_2$-X-$(CH_2)_n$-$R_1$ oder den Rest

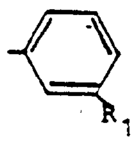

n 1 oder 3,

$R_1$ den Rest

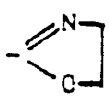 , den Rest 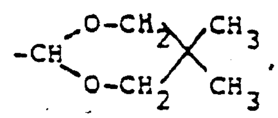

$—COCH_3$, $COOR_2$, wobei $R_2$ Wasserstoff oder gegebenenfalls durch Halogen, Phenyl, $C_1$-$C_4$-Alkoxy oder $C_1$-$C_4$-Dialkyl-amino substituiertes Alkyl mit 1-10 C-Atomen, Cycloalkyl, Aryl oder einen heterocyclischen Rest bedeuten kann, oder den Rest $CONHR_3$ mit $R_3$ in der Bedeutung Wasserstoff oder eines Alkanoyl- oder Alkansulfonylrestes mit je 1-10 C-Atomen darstellt,

$R_9$ den Rest —Z-$(CH_2)_m$-$R_6$

m = 2-10,

Z eine —C≡C—, eine cis-CH=CH— oder eine trans-CH=CH— Gruppe bedeutet,

$R_6$ $N_3$, Cl, Br, J oder $COOR'_2$,

$R'_2$ Wasserstoff oder eine Methyl- oder Ethylgruppe,

X ein Sauerstoffatom oder eine Methylengruppe,

$Y_2$ Wasserstoff oder Fluor,

A eine $—CH_2$-$CH_2—$, trans-CH=CH— oder —C≡C-Gruppe,

W eine freie oder funktionell abgewandelte Hydroxymethylengruppe oder eine freie oder funktionell abgewandelte

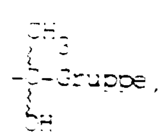

wobei die OH Gruppe α- oder β-ständig sein kann,

D die Gruppe

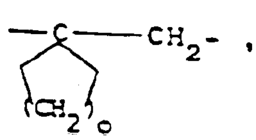

eine geradkettige, gesättigte Alkylengruppe mit 1-5 C-Atomen, eine verzweigte gesättigte oder eine geradkettige oder verzweigte ungesättigte Alkylengruppe mit 2-5 C-Atomen, die gegebenenfalls durch

Fluoratome substituiert sein können,

o 1, 2 oder 3 bedeutet,

E eine Direktbindung, eine —C≡C-Gruppe oder eine —CH=CR$_7$-Gruppe darstellt, wobei R$_7$ ein Wasserstoffatom, eine Alkylgruppe mit 1-5 C-Atomen oder Halogen bedeuten,

R$_4$ eine Alkylgruppe mit 1-10 C-Atomen, eine Cycloalkylgruppe mit 3-10 C-Atomen oder eine gegebenenfalls substituierte Arylgruppe mit 6-10 C-Atomen oder eine heterocyclische Gruppe,

R$_5$ eine freie oder funktionell abgewandelte Hydroxygruppe, und, falls R$_2$ die Bedeutung eines Wasserstoffatoms hat, deren Salze mit physiologisch verträglichen Basen bedeuten und deren Cyclodextrinclathrate.

2. Verfahren zur Herstellung von Carbacyclinderivaten, der allgemeinen Formel I, dadurch gekennzeichnet, daß man in an sich bekannter Weise a) eine Verbindung der Formel IV,

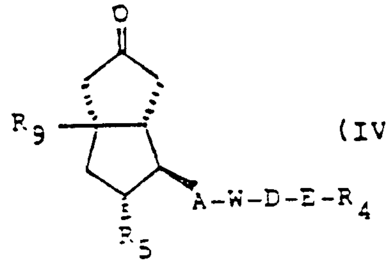

(IV),

worin A, W, D, E, R$_4$, R$_5$ und R$_9$ die bereits angegebenen Bedeutungen haben, mit einem Wittigreagenz der allgemeinen Formeln V oder VI oder einem Dianion der Formel VII

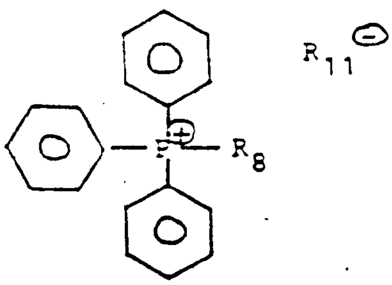

(V)

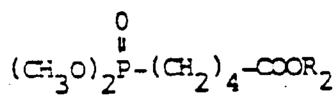

(VI)

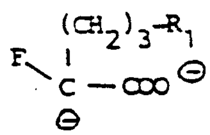

(VII),

worin

R$_2$ die genannte Bedeutung und R$_8$ die Reste —CH$_2$-CH$_2$-X-(CH$_2$)$_n$-R$_1$ oder

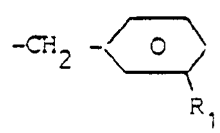

mit den bereits erwähnten Bedeutungen für X, n und R$_1$ und R$_{11}$ Brom oder Chlor bedeuten, in Gegenwart von K -tert.-Butylat umsetzt oder b) eine Verbindung der Formel VIII, die man aus dem entsprechenden 4-Ester durch DIBAH-Reduktion erhält,

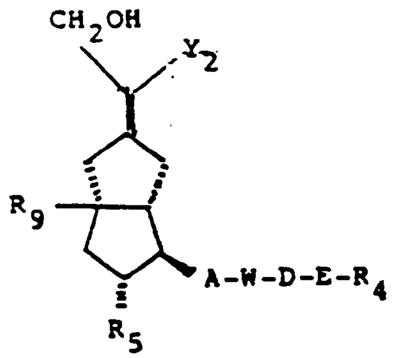

(VIII),

worin A, W, D, E, $R_4$, $R_5$, $R_9$ und $Y_2$ die bereits angegebenen Bedeutungen haben, gegebenenfalls nach Schutz anwesender freier Hydroxy- oder Aminogruppen mit einem Halogenalkansäurederivat der Formel IX

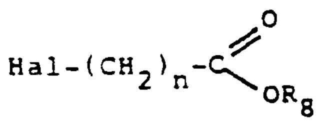

(IX),

wobei n 1 oder 3, Hal ein Chlor- oder Bromatom und $R_8$ einen Alkylrest mit 1-4 C-Atomen oder ein Alkalimetall bedeuten, in Gegenwart einer Base verethert und gegebenenfalls anschließend in beliebiger Reihenfolge Isomere trennt und/oder geschützte Hydroxygruppen freisetzt und/oder freie Hydroxygruppen verestert, verethert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

3. Arzneimittel, enthaltend ein oder mehrere Verbindungen gemäß Anspruch 1 und übliche Hilfs- und Trägerstoffe.

4. 5(Z)-{1(S,β)-[5-Azido-1-pentinyl]-6β-[(E)-3α-hydroxy-4-methyl-oct-6-in-1-enyl]-7α-hydroxy-bicyclo [3.3.0] octan-3-yliden} -pentansäure.

5. 5(E/Z)-{1(S,β)-[4-Carboxy-1-butinyl]-6β-[(E)-3α-hydroxy-4-methyl-oct-6-in-1-enyl]-7α-hydroxy-bicyclo [3.3.0] octan-3-yliden}-pentansäuremethylester.

6. 5(E/Z)-{1(S,β)-[4-Carboxy-1-butinyl]-6β-[(E)-3α-hydroxy-4-methyl-oct-6-in-1-enyl]-7α-hydroxy-bicyclo [3.3.0] octan-3-yliden}-pentansäure.

7. 5(E)-{1(S,β)-[5-Hydroxy-1-pentinyl]-6β-[(E)-3α-(tetrahydropyran-2-yl-oxy)-4-methyl-oct-6-in-1-enyl]-7α-(tetrahydropyran-2-yl-oxy)-bicyclo [3.3.0] octan-3-yliden}-pentansäuremethylester.

## Claims

1. Carbacyclin derivatives of formula I

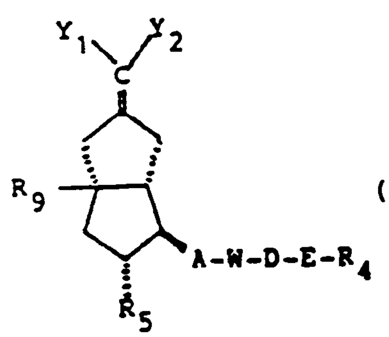

(I),

wherein

$Y_1$ represents the radical $—CH_2\text{-}X\text{-}(CH_2)_n\text{-}R_1$ or the radical

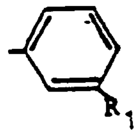

n is 1 or 3,

$R_1$ represents the radical

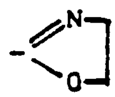
,

the radical

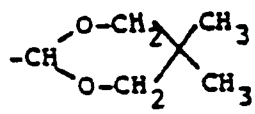
,

—$COCH_3$, $COOR_2$ wherein $R_2$ may represent hydrogen or alkyl having from 1 to 10 carbon atoms (optionally substituted by halogen, phenyl, $C_1$-$C_4$-alkoxy or by $C_1$-$C_4$-dialkylamino), cycloalkyl, aryl or a heterocyclic radical, or the radical $CONHR_3$ wherein $R_3$ represents hydrogen or an alkanoyl or alkanesulphonyl radical each having from 1 to 10 carbon atoms,

$R_9$ represents the radical —Z-$(CH_2)_m$-$R_6$,

m = 2-10,

Z represents a —C≡C—, a cis-CH=CH— or a trans-CH=CH— group,

$R_6$ represents $N_3$, Cl, Br, I or $COOR'_2$,

$R'_2$ represents hydrogen or a methyl or ethyl group,

X represents an oxygen atom or a methylene group,

$Y_2$ represents hydrogen or fluorine,

A represents a —$CH_2$-$CH_2$—, trans-CH=CH— or —C≡C— group,

W represents a free or functionally modified hydroxymethylene group or a free or functionally modified

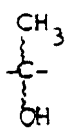

group in which the OH group may be in the α- or β-configuration,

D represents the group

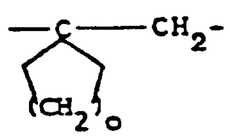
,

a straight-chain, saturated alkylene group having from 1 to 5 carbon atoms, a branched saturated or a straight-chain or branched unsaturated alkylene group having from 2 to 5 carbon atoms, which may optionally be substituted by fluorine atoms,

o is 1, 2 or 3,

E represents a direct bond, a —C≡C— group or a —CH=$CR_7$— group wherein $R_7$ represents a hydrogen atom, an alkyl group having from 1 to 5 carbon atoms or halogen,

$R_4$ represents an alkyl group having from 1 to 10 carbon atoms, a cycloalkyl group having from 3 to 10 carbon atoms, an optionally substituted aryl group having from 6 to 10 carbon atoms or a heterocyclic group,

$R_6$ represents a free or functionally modified hydroxy group, and, if $R_2$ represents a hydrogen atom, the salts thereof with physiologically tolerable bases, and the cyclodextrin clathrates thereof.

2. Process for the preparation of carbacyclin derivatives of the general formula I, characterised in that, in

a manner known per se, a) a compound of formula IV

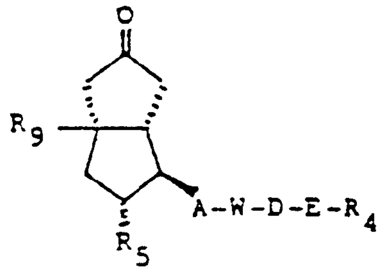

(IV),

wherein A, W, D, E, $R_4$, $R_5$ and $R_9$ have the meanings already given, is reacted with a Wittig reagent of the general formulae V or VI or a dianion of formula VII

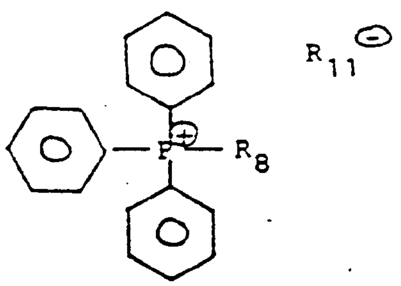

(V)

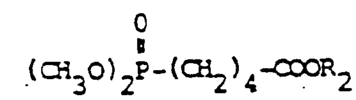

(VI)

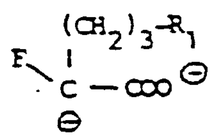

(VII),

wherein

$R_2$ has the meaning given and $R_5$ represents the radical —$CH_2$-$CH_2$-X-$(CH_2)_n$-$R_1$ or

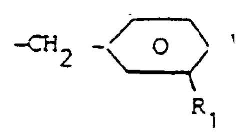

with the meanings already mentioned for X, n and $R_1$, and $R_{11}$ represents bromine or chlorine, in the presence of potassium tert.-butoxide, or b) a compound of formula VIII, which is obtained from the corresponding 4-ester by DIBAH reduction,

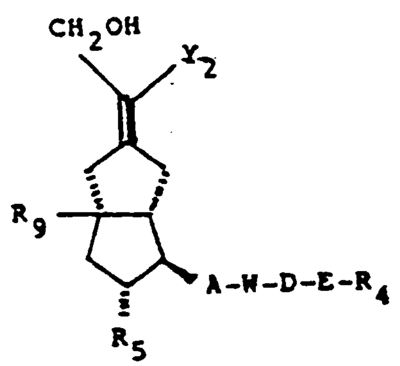

(VIII),

wherein A, W, D, E, $R_4$, $R_5$, $R_9$ and $Y_2$ have the meanings already given, is etherified in the presence of a base, optionally after the protection of any free hydroxy or amino groups present, with a haloalkanoic acid derivative of formula IX

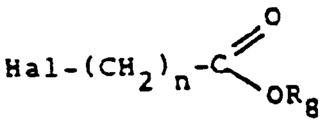

(IX),

wherein n is 1 or 3, Hal represents a chlorine or bromine atom and $R_8$ represents an alkyl radical having from 1 to 4 carbon atoms, or an alkali metal, and optionally then, in any order, isomers are separated and/or protected hydroxy groups are freed and/or free hydroxy groups are esterified or etherified and/or a free carboxy group is esterified and/or an esterified carboxy group is hydrolysed or a carboxy group is converted into an amide or converted with a physiologically tolerable base into a salt.

3. Medicament, comprising one or more compounds according to claim 1 together with customary auxiliaries and carriers.

4. 5(Z)-(1(S,β)-[5-azido-1-pentynyl]-6β-[(E)-3α-hydroxy-4-methyl-oct-6-yn-1-enyl]-7α-hydroxybicyclo[3.3.0] octan-3-ylidene}-pentanoic acid.

5. 5(E/Z)-{1(S,β)-[4-carboxy-1-butynyl]-6β-[(E)-3α-hydroxy-4-methyl-oct-6-yn-1-enyl]-7a-hydroxybicyclo-[3.3.0] octan-3-ylidene}-pentanoic acid methyl ester.

6. 5(E/Z)-{1(S,β)-[4-carboxy-1-butynyl]-6β-[(E)-3α-hydroxy-4-methyl-oct-6-yn-1-enyl]-7a-hydroxybicyclo-[3.3.0] octan-3-ylidene}-pentanoic acid.

7. 5(E)-{1(S,β)-[5-hydroxy-1-pentynyl]-6β-[(E)-3α-(tetrahydropyran-2-yloxy)-4-methyl-oct-6-yn-1-enyl]-7α-(tetrahydropyran-2-yloxy)-bicyclo[3.2.0] octan-3-ylidene}-pentanoic acid methyl ester.

## Revendications

1. Dérivés de carbacyclines de formule I

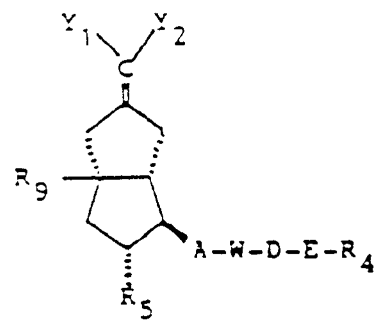

(I),

dans laquelle

$Y_1$ représente le reste —$CH_2X$-$(CH_2)_n$-$R_1$ ou le reste

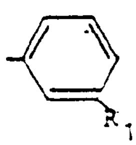

n vaut 1 ou 3,

$R_1$ représente le reste

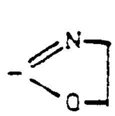

, le reste

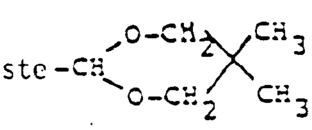

,

un groupe —$COCH_3$, $COOR_2$, où $R_2$ représente un atome d'hydrogène ou un groupe alkyle ayant 1 à 10 atomes de carbone, (éventuellement substitué par de l'halogène, par un groupe phényle, par un

groupe alcoxy en $C_1$ à $C_4$, par un groupe dialkylamino en $C_1$ à $C_4$) cycloalkyle ou aryle ou peut représenter un reste hétérocyclique, ou bien il représente le reste $CONHR_3$ dans lequel $R_3$ représente un atome d'hydrogène ou un reste alcanoyl ou alcane sulfonyle ayant chacun 1 à 10 atomes de carbone, $R_9$ représente le reste —Z-$(CH_2)_m$ -$R_6$,

m vaut 2 à 10,

Z représente un groupe C≡C—, un groupe cis-CH=CH— ou un groupe trans-CH=CH—,

$R_6$ représente $N_3$, Cl, Br, I ou $COOR'_2$,

$R'_2$ représente un atome d'hydrogène ou un groupe méthyle ou éthyle,

X représente un atome d'oxygène ou un groupe méthylène,

$Y_2$ représente un atome d'hydrogène ou de fluor,

A représente un groupe $CH_2$-$CH_2$—, trans-CH=CH— ou C≡C—,

W représente un groupe hydroxyméthylène libre ou dont le groupe fonctionnel est modifié, ou un groupe

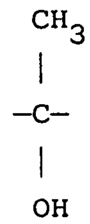

libre ou un groupe fonctionnel modifié, le groupe OH pouvant être en position α ou β,

D représente le groupe

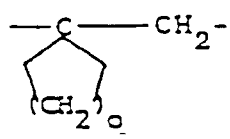 ,

un groupe alkylène linéaire et saturé comportant 1 à 5 atomes de carbone, un groupe alkylène (ayant 1 à 5 atomes de carbone, saturé, linéaire), un groupe alkylène (ayant 2 à 5 atomes de carbone, ramifié et saturé ou linéaire ou ramifié et insaturé) qui peuvent également être substitués par des atomes de fluor,

o vaut 1, 2 ou 3,

E représente une liaison directe, un groupe —C≡C— ou un groupe —CH=$CR_7$—, où $R_7$ représente un atome d'hydrogène, un groupe alkyle ayant 1 à 5 atomes de carbone ou un atome d'halogène,

$R_4$ représente un groupe alkyle ayant 1 à 10 atomes de carbone, un groupe cycloalkyle ayant 3 à 10 atomes de carbone ou un groupe aryle, éventuellement substitué et comportant 6 à 10 atomes de carbone, ou un groupe hétérocylique,

$R_6$ représente un groupe hydroxyle libre ou modifié, et, si $R_2$ représente un atome d'hydrogène, ses sels avec des bases physiologiquement acceptables, et leurs complexes d'inclusion avec des cyclodextrines.

2. Procédé pour préparer des dérivés de carbacyclines de formule générale I, procédé caractérisé en ce que, de façon connue en soi (a) on fait réagir un composé de formule IV

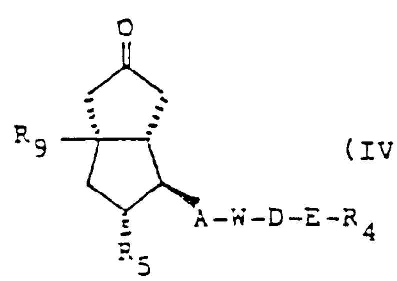 (IV),

(dans laquelle A, W, D, E, $R_4$, $R_6$ et $R_9$ ont les sens déjà indiqués) avec un réactif de Wittig répondant aux formules générales V et VI ou avec un dianion de formule VII

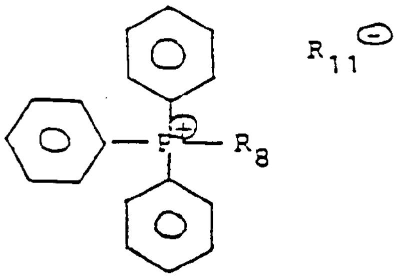 (V) 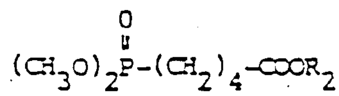 (VI)

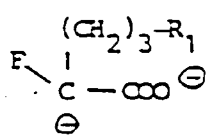 (VII),

(formules dans lesquelles $R_2$ a le sens indiqué et $R_8$ représente les restes $—CH_2-CH_2-X-(CH_2)_n-R_1$ ou

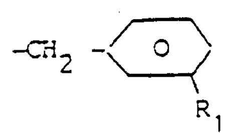

avec les sens déjà indiqués pour X, n et $R_1$, et $R_{11}$ représente un atome de brome ou de chlore), en présence de tertiobutylate de potassium, ou bien b) on soumet un composé de formule (VIII), que l'on obtient par réduction de DIBAH de l'ester – 4 correspondant :

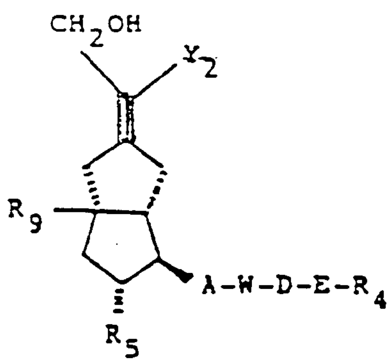 (VIII),

(dans laquelle A, W, D, E, $R_4$, $R_5$, $R_9$ et Y ont le sens déjà indiqué), éventuellement après avoir protégé des groupes hydroxy et amino libres présents, à une éthérification à l'aide d'un dérivé d'un acide halogénoalcanoïque de formule IX

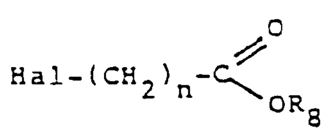 (IX),

(dans laquelle n vaut 1 ou 3 ; Hal représente un atome de chlore ou de brome, et $R_5$ représente un reste alkyle ayant 1 à 4 atomes de carbone ou un métal alcalin) en présence d'une base et, éventuellement, on effectue ensuite dans un ordre quelconque une séparation des isomères et/ou on met en liberté des groupes hydroxyles protégés et/ou on estérifie ou éthérifie des groupes hydroxyles libres et/ou on estérifie un groupe carboxyle libre et/ou on saponifie un groupe carboxyle estérifié ou l'on transforme un groupe carboxyle en un groupe amide ou, avec une base physiologiquement tolérable, en un sel.

3. Médicament, contenant un ou plusieurs composés selon la revendication 1 et des adjuvants et excipients ou supports usuels.

4. L'acide (Z){(S,β)-[azido-5 pentynyl-1]-1 [(E) hydroxy-3α méthyl-4 octyn-6 ényl-1]-6β hydroxy-7α bicyclo [3.3.0] octanylidène-3} 5 pentanoïque.

5. Ester méthylique de l'acide (E/Z) {(S,β) [carboxy-4 butynyl-1]-1-[(E)hydroxy-3α méthyl-4 octyn-6 ényl-1]-6β hydroxy-7α bicyclo [3.3.0] octanylidène-3}-5 pentanoïque.

6. Acide {(S,β)[carboxy-4 butynyl-1]-1[(E) hydroxy-3α méthyl-4 octyn-6 ényl-1]-6β hydroxy-7α bicyclo[3.3.0] octanylidène-3}-5 (E/Z) pentanoïque.

7. Ester méthylique de l'acide (E) {(S,β) [hydroxy-5 pentynyl-1]-1[(E)(tétrahydropyrannyl-2 oxy)-3α méthyl-4 octyn-6 ényl-1]-6β (tétrahydropyrannyl-2 oxy)-7α bicyclo [3.3.0] octanylidène-3}-5E pentanoïque.